# EUROPEAN PATENT SPECIFICATION

(11) **EP 3 915 501 B1**
(45) Date of publication and mention of the grant of the patent: **14.09.2022**
(21) Application number: 20199230.2
(22) Date of filing: 30.09.2020
(51) Int. Cl.: A61B 18/14, A61B 18/00

(54) **INTRALUMINAL REFERENCE ELECTRODE FOR CARDIOVASCULAR TREATMENT APPARATUS**
INTRALUMINALE REFERENZELEKTRODE FÜR KARDIOVASKULÄRE BEHANDLUNGSVORRICHTUNG
ÉLECTRODE DE RÉFÉRENCE INTRALUMINALE POUR APPAREIL DE TRAITEMENT CARDIOVASCULAIRE

(30) Priority: 29.05.2020 US 202063031955 P; 16.07.2020 US 202063052553 P; 23.09.2020 US 202017029890; 23.09.2020 US 202017029752
(43) Date of publication of application: 01.12.2021
(73) Proprietor: Biosense Webster (Israel) Ltd., Yokneam 2066717 (IL)
(72) Inventor: VAN NIEKERK, Pieter Emmelius, Irvine, CA 92618 (US); MALINARIC, Jamie Lynn, Irvine, CA 92618 (US); FUENTES-ORTEGA, Cesar, Irvine, CA 92618 (US); SALAZAR, Henry, Irwindale, CA 91706 (US); TOBEY, Dustin R., Irwindale, CA 91706 (US); BASU, Shubhayu, Irwindale, CA 91706 (US); THALER, Sean D., Irwindale, CA 91706 (US); WONG, Joseph R., Irwindale, CA 91706 (US); GHIDOLI, Daniele, Irwindale, CA 91706 (US)
(74) Representative: Carpmaels & Ransford LLP

(56) References cited:
- US-A1- 2014 316 294
- US-A1- 2019 350 649
- US-A1- 2020 129 125

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy and/or electroporation), it may be possible to cease or modify the propagation of unwanted electrical signals from one portion of the heart to another. The ablation process may provide a barrier to unwanted electrical pathways by creating electrically insulative lesions or scar tissue.

In some procedures, a catheter with one or more electrodes can be used to provide ablation within the cardiovascular system. The catheter can be inserted into a major vein or artery (e.g., the femoral artery) and then advanced to position the electrodes within the heart or in a cardiovascular structure adjacent to the heart (e.g., the pulmonary vein). The electrodes can be placed in contact with cardiac tissue or other vascular tissue and then electrically activated to ablate the contacted tissue. In some cases, the electrodes can be bipolar. In some other cases, a monopolar electrode may be used in conjunction with a ground pad that is in contact with the patient.

Examples of ablation catheters are described in U.S. Pub. No. 2013/0030426, entitled "Integrated Ablation System using Catheter with Multiple Irrigation Lumens," published Jan. 31, 2013; U.S. Pat. No. 10,660,700 entitled "Irrigated Balloon Catheter with Flexible Circuit Electrode Assembly," issued May 26, 2020; U.S. Pub. No. 2018/0071017, entitled "Ablation Catheter with a Flexible Printed Circuit Board," published Mar. 15, 2018; U.S. Pub. No. 2018/0056038, entitled "Catheter with Bipole Electrode Spacer and Related Methods," published Mar. 1, 2018; U.S. Pub. No. 2018/0036078, entitled "Catheter with Soft Distal Tip for Mapping and Ablating Tubular Region," published Feb. 8, 2018; U.S. Pat. No. 8,956,353, entitled "Electrode Irrigation Using Micro-Jets," issued Feb. 17, 2015; and U.S. Pat. No. 9,801,585, entitled "Electrocardiogram Noise Reduction," issued Oct. 31, 2017, for each of which a copy is provided in the priority U.S. Provisional Patent Application 63/052,553.

Some catheter ablation procedures may be performed using electrophysiology (EP) mapping. Such EP mapping may include the use of sensing electrodes on a catheter (e.g., the same catheter that is used to perform the ablation). The sensing electrodes can be the same or different electrodes as those used to perform ablation. The sensing electrodes can monitor electrical signals within the cardiovascular system to pinpoint the location of aberrant conductive tissue sites that are responsible for the arrhythmia. Examples of an EP mapping system are described in U.S. Pat. No. 5,738,096, entitled "Cardiac Electromechanics," issued Apr. 14, 1998 of which a copy is provided in the priority U.S. Provisional Patent Application 63/052,553. Examples of EP mapping catheters are described in U.S. Pat. No. 9,907,480, entitled "Catheter Spine Assembly with Closely-Spaced Bipole Microelectrodes," issued Mar. 6, 2018; U.S. Pat. No. 10,130,422, entitled "Catheter with Soft Distal Tip for Mapping and Ablating Tubular Region," issued Nov. 20, 2018; and U.S. Pub. No. 2018/0056038, entitled "Catheter with Bipole Electrode Spacer and Related Methods," published Mar. 1, 2018, for each of which a copy is provided in the priority U.S. Provisional Patent Application 63/052,553.

EP mapping procedures may include the use of a reference electrode on a mapping catheter to sense electrical potentials in fluids near tissue. An example of such reference electrode is described in U.S. Pub. No. 2020/0038101, entitled "Unipolar reference electrode for electrophysiology mapping catheter," published Feb. 6, 2020, of which a copy is provided in the priority U.S. Provisional Patent Application 63/052,553.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO system by Biosense Webster, Inc. of Irvine, Calif. Examples of catheters that are configured for use with an IGS system are disclosed in U.S. Pat. No. 9,480,416, entitled "Signal Transmission Using Catheter Braid Wires," issued Nov. 1, 2016, of which a copy is provided in the priority U.S. Provisional Patent Application 63/052,553; and various other references that are cited herein and attached in the Appendix in the priority U.S. Provisional Patent Application 62/052,553.

### SUMMARY

Examples presented herein generally include systems and methods which can contact electrodes to cardiac tissue while sensing electrical potential from fluids near the tissue. The electrodes in contact with the tissue can be carried by an end effector. The end effector sensors can be configured to ablate and/or sense electrical potentials from the cardiac tissue in which they are in contact. A reference electrode can be insulated by a shaft connected to the end effector so that the reference electrode can sense the electrical potentials from the fluids near the tissue while being prevented, by the shaft geometry, from contacting the cardiac tissue. The reference electrode can further serve as an extension of an irrigation tube positioned to irrigate the treatment area.

An example apparatus can include an elongated shaft, an end effector, and a reference electrode. A proximal portion of the elongated shaft can be manipulated to position a distal portion of the elongated shaft into a heart of a patient. The end effector can be disposed near the distal portion of the elongated shaft and can include spines each carrying at least one spine electrode. The spine electrodes can be positioned and otherwise configured to contact cardiovascular tissue. The spine electrodes can further be configured to receive electrical potentials from the tissue. The reference electrode can include a tubular, electrically conductive inner surface and an outer surface. The reference electrode can be disposed in the elongated shaft such that the electrode does not protrude beyond the elongated shaft. The inner surface can receive electrical potentials from fluids so that the electrical potentials received by the reference electrode act as a referential signal for the electrical potentials of tissue received by the electrodes carried by the spines. The outer surface of the reference electrode can be substantially electrically isolated from the electrical potentials of the fluids by an electrically insulating cover.

The apparatus can further include a connector joining the elongated shaft to the end effector. The connector can include the electrically insulating cover which electrically isolates the outer surface of the reference electrode.

The reference electrode can have a distal end approximately coplanar or coaxial to a distal end of the connector. The connector can be a distal extension of the shaft such that the reference electrode is approximately coplanar or coaxial with a distal end of the shaft by virtue of being approximately coplanar or coaxial with the distal end of the connector.

The connector can be capable of contacting the cardiovascular tissue while the spine electrodes are in contact with the cardiovascular tissue and while the reference electrode is electrically insulated, by the connector, from the cardiovascular tissue.

The end effector can have an unconstrained configuration aligned with a longitudinal axis of the apparatus and a particular geometry in the unconstrained configuration. The connector can include a tubular outer surface with a diameter measured orthogonal to the longitudinal axis. The end effector can have a height measured orthogonal to the longitudinal axis and a width measured orthogonal to the height and longitudinal axis when the end effector is in the unconstrained configuration. The height of the end effector can measure approximately equal to or less than the diameter of the tubular outer surface of the connector, and the width of the end effector can measure greater than the diameter of the tubular outer surface of the connector.

The apparatus can further include an irrigation tube in fluidic communication with the inner surface of the reference electrode. The reference electrode can function as a distal extension of the irrigation tube. The irrigation tube can be positioned within a lumen of the reference electrode and/or around the outer surface of the reference electrode. In addition, or as an alternative to the outer surface being electrically insulated by the connector joining the elongated shaft to the end effector, the outer surface of the reference electrode can be electrically insulated by a portion of the irrigation tube positioned over the outer surface of the reference electrode.

The spine electrodes can be positioned to preferably be distributed in a radially asymmetric pattern on the cardiovascular tissue in relation to the reference electrode's position when the plurality of spine electrodes are in contact with the cardiovascular tissue.

The spine electrodes can be positioned to form a rectangular grid when the plurality of spine electrodes are contact with the cardiovascular tissue.

The end effector can have a particular geometry including three loop members. Each of the loop members can include at least one of the spines (which each carry electrode positioned to contact tissue). The loop members can be joined at a common distal vertex. When the end effector is unconstrained, the distal vertex is aligned with a longitudinal axis defined by the elongated shaft. Each of the three loop members can include a respective pair of ends affixed to the distal portion of the elongated shaft. The end effector can deflect away from the unconstrained configuration at an angle relative to the longitudinal axis to position the spine electrodes against a planar surface.

The connector can secure each of the respective pair of ends of the loop members to the distal portion of the elongated shaft.

The end effector can deflect at an angle relative to the longitudinal axis to position the spine electrodes against a planar area of the cardiovascular tissue.

The spine electrodes can receive electrical potentials from the cardiovascular tissue when in contact with the cardiovascular tissue.

The spine electrodes can ablate when in contact with the cardiovascular tissue.

An example method can include one or more of the following steps presented in no particular order. An end effector can be assembled such that the end effector carries at least one spine electrode thereon that is configured to contact cardiovascular tissue and receive electrical potentials from the tissue. A reference electrode can be affixed in relation to the end effector such that a tubular inner surface of the reference electrode is configured to receive electrical potentials from fluids so that the electrical potential from the fluids act as a referential signal for the electrical potentials received by the spine electrodes. An outer surface of the reference electrode can be electrically insulated such that the outer surface is electrically isolated from the electrical potentials from fluids approximate the cardiovascular tissue while the inner surface is capable of receiving the electrical potentials of the fluids. The end effector and reference electrode can be affixed to a distal portion of an elongated shaft, the elongated shaft configured to be manipulated at a proximal portion of the elongated shaft to position the distal portion into a heart of a patient.

The method can further include joining the elongated shaft to the end effector with a connector and electrically insulating the outer surface of the reference electrode with the connector. The method can further include joining the end effector with the connector and an outer surface area of the reference electrode partially exposed electrode and positioned without the possibility of touching tissue.

The method can further include affixing the reference electrode and the connector such that a distal end of the reference electrode and a distal end of the connector are approximately coplanar and coaxial to each other. The connector can be a distal extension of the shaft such that the reference electrode is approximately coplanar or coaxial to the distal end of the shaft by virtue of being approximately coplanar or coaxial to the distal end of the connector.

The method can further include joining the elongated shaft to the end effector such that the connector is capable of contacting the cardiovascular tissue while the spine electrode is in contact with the cardiovascular tissue and while the reference electrode is electrically insulated, by the connector, from the cardiovascular tissue.

The method can further include assembling the end effector such that the end effector has an unconstrained configuration, and such that, when in the unconstrained configuration, the end effector has a height that is approximately equal to or less than a diameter of a tubular outer surface of the connector and the end effector has a width measuring greater than the diameter of the tubular outer surface of the connector, the diameter and height being measured orthogonal to a longitudinal axis defined by the elongated shaft, and the width being measured orthogonal to the longitudinal axis and direction of measurement of the height.

The method can further include positioning an irrigation tube within the elongated shaft such that the irrigation tube is in fluidic communication with the inner surface of the reference electrode.

The method can further include affixing the reference electrode to the irrigation tube such that the reference electrode is a distal extension of the irrigation tube.

The method can further include arranging the spine electrodes in a rectangular grid. Alternatively, the method can further include arranging the spine electrodes in a non-rectangular grid.

The method can further include shaping a first loop member, a second loop member and a third loop member to each form a respective loop; coupling a respective pair of ends for each of the first loop member, the second loop member, and the third loop member to the distal portion of the elongated shaft; joining the first loop member, the second loop member, and the third loop member at a common distal vertex distal to the distal portion of the elongated shaft; and configuring the end effector to be capable of deflecting at an angle relative to a longitudinal axis defined by the elongated shaft to position the spine electrodes against a planar surface.

The method can further include securing each of the respective pairs of ends to the distal portion of the elongated shaft with a connector and electrically insulating the outer surface of the reference electrode with the connector.

The method can further include configuring the end effector to deflect at an angle relative to the longitudinal axis to position the spine electrodes against a planar area of the cardiovascular tissue.

Another example method can include one or more of the following steps presented in no particular order. Steps of this example method are combinable with steps of the previous example method. A distal portion of the elongated shaft, and an end effector extending distally from a distal portion of the elongated shaft, can be moved through a catheter to a heart. The end effector can be moved from a distal end of the catheter via manipulation of a proximal portion of the elongated shaft. Spine electrodes, carried by the end effector, can be juxtaposed to cardiovascular tissue via manipulation of the proximal portion of the elongated shaft. While the spine electrodes are opposed to the cardiovascular tissue, electrical potentials from fluids approximate the cardiovascular tissue can be received via an inner surface of a reference electrode, an outer surface of the reference electrode being electrically insulated from the electrical potentials.

The method can further include contacting, to the cardiovascular tissue, while the spine electrodes are opposed (i.e., in physical contact) to the cardiovascular tissue, a connector joining the end effector to the distal portion of the elongated shaft and electrically insulating the outer surface of the reference electrode from the electrical potentials.

The method can further include irrigating the cardiovascular tissue through a lumen defined by the inner surface of the reference electrode.

The method can further include positioning the spine electrodes in a pattern on the cardiovascular tissue that is radially asymmetric in relation to the reference electrode's position.

The method can further include positioning the spine electrodes in a rectangular grid on the cardiovascular tissue.

The method can further include deflecting the end effector at an angle relative to a longitudinal axis defined by the elongated shaft to position the spine electrodes against the cardiovascular tissue. The cardiovascular tissue against which the spine electrodes are positioned being can be substantially planar.

The method can further include receiving, while the spine electrodes are opposed to the cardiovascular tissue, electrical potentials from the cardiovascular tissue via the spine electrodes.

The method can further include ablating the cardiovascular tissue via the spine electrodes, while the spine electrodes are opposed to the cardiovascular tissue.BRIEF

### DESCRIPTION OF THE DRAWINGS

Figure 1 is an illustration of a cardiovascular treatment apparatus having an end effector at a distal portion of an elongated shaft and a proximal handle at a proximal portion of the elongated shaft according to aspects of the present invention.
Figures 2A and 2B are illustrations of cross sectional views of an intermediate section and distal portion of a shaft of the apparatus in greater detail according to aspects of the present invention.
Figures 3A through 3C are various views of the end effector and distal portion of the shaft according to aspects of the present invention.
Figures 3D through 3F are illustrations of various views of the distal portion of the shaft according to aspects of the present invention.
Figures 4A through 4C are illustrations of the end effector being pressed to a planar surface according to aspects of the present invention.
Figures 5A and 5B are illustrations of cross sectional views of alternative reference electrode configurations according to aspects of the present invention.
Figure 6 is an illustration of a treatment utilizing the cardiovascular treatment apparatus according to aspects of the present invention.
Figures 7A through 7F are illustrations of alternative end effectors having loop members and including an intralumenal reference electrode according to aspects of the present invention.
Figure 8 is an illustration of an alternative end effector having a ray geometry and including an intralumenal reference electrode according to aspects of the present invention.
Figure 9 is an illustration of another alternative end effector including an intralumenal reference electrode according to aspects of the present invention.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which includes by way of illustration, one of the best modes contemplated for carrying out the invention. As will be realized, the invention is capable of other different or equivalent aspects, all without departing from the invention, as defined by the appended claims. Accordingly, the drawings and descriptions should be regarded as illustrative in nature and not restrictive.

Teachings, expressions, versions, examples, etc. described herein may be combined with other teachings, expressions, versions, examples, etc. that are described herein, including those examples provided in the references attached in the Appendix hereto. The following-described teachings, expressions, versions, examples, etc. should therefore not be viewed in isolation relative to each other. Various suitable ways in which the teachings herein may be combined are apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

As used herein, the term "non-transitory computer-readable media" includes, but is not limited to, random access memory (RAM), read-only memory (ROM), electronically erasable programmable ROM (EEPROM), flash memory or other memory technology, compact disc ROM (CD-ROM), digital versatile disks (DVD) or other optical storage, magnetic cassettes, magnetic tape, magnetic disk storage or other magnetic storage devices, or any other tangible, physical medium which can be used to store computer readable information.

As used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

As used herein, the term "wire" can include elongated solid core and hollow core structures. When used to refer to an electrical conductor, the term wire "wire" can include insulated, non-insulated, individual, bundled, and integrated circuit conductors.

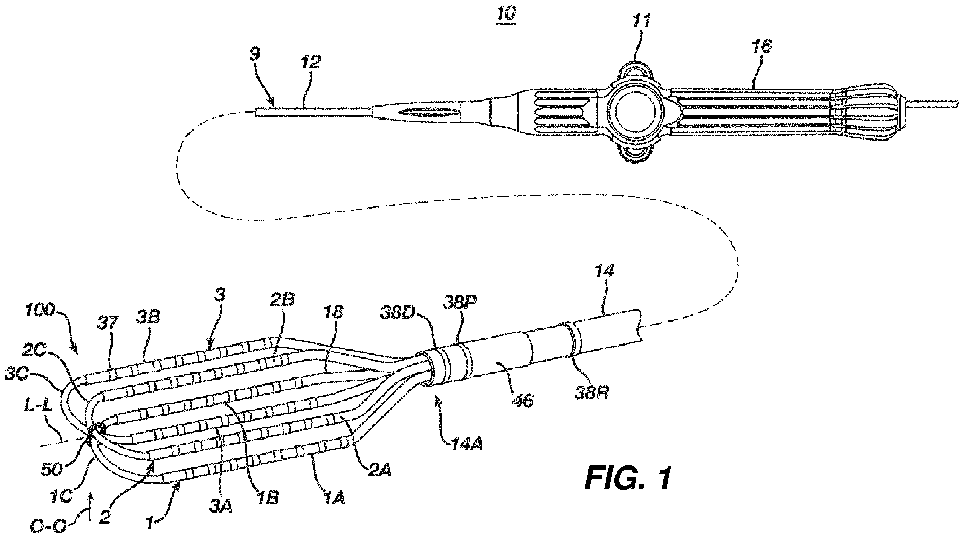

Figure 1 illustrates an example apparatus 10 having an elongated shaft 9, a distal electrode assembly or end effector 100, and a deflection control handle 16. The apparatus 10 can have several design variations while including novel aspects illustrated herein. The apparatus 10 is presented for illustration purposes only and is not intended to be limiting. Details of the apparatus can be understood with reference to U.S. Patent Application S.N. 63/031,955 to which the present application claims the benefits of priority.

The elongated shaft 9 has a proximal portion 12 in the shape of an elongated catheter body, an intermediate deflection section 14, and distal portion 14A. The deflection control handle 16 is attached to the proximal end of the catheter body 12. The distal portion 14A of the shaft is coupled to the end effector 100 via a connector tubing 46. The connector tubing 46 is considered herein to be a distal extension of the elongated shaft such that the connector tubing 46 is considered a part of the elongated shaft 9. The elongated shaft 9 forms a tubular catheter body sized and otherwise configured to traverse vasculature. The proximal portion 12 of the elongated shaft 9 can be manipulated (e.g. via the handle 16) to position the distal portion 14A of the shaft 9 into a heart of a patient. The end effector 100 has a plurality of loop members 1, 2, 3 that overlap at a common distal vertex 50. The loop members 1, 2, 3 can be joined at the distal vertex 50 by a mechanical linkage.

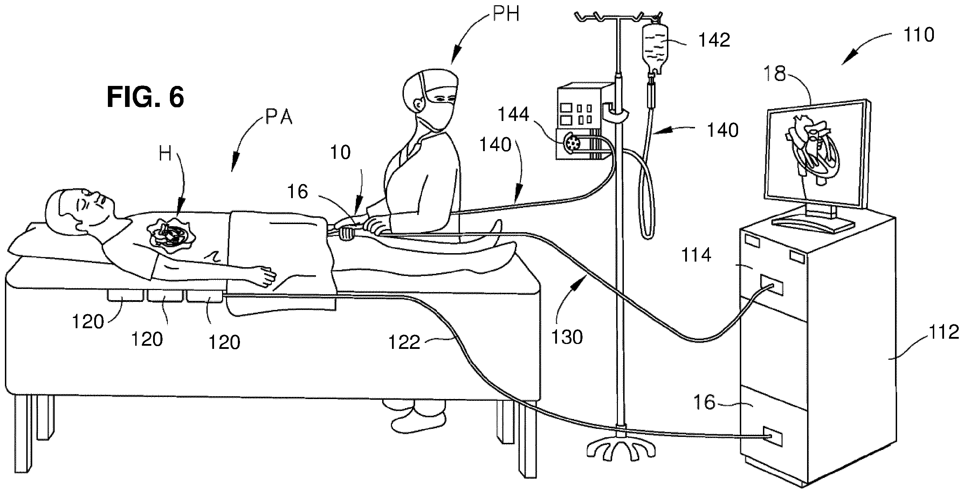

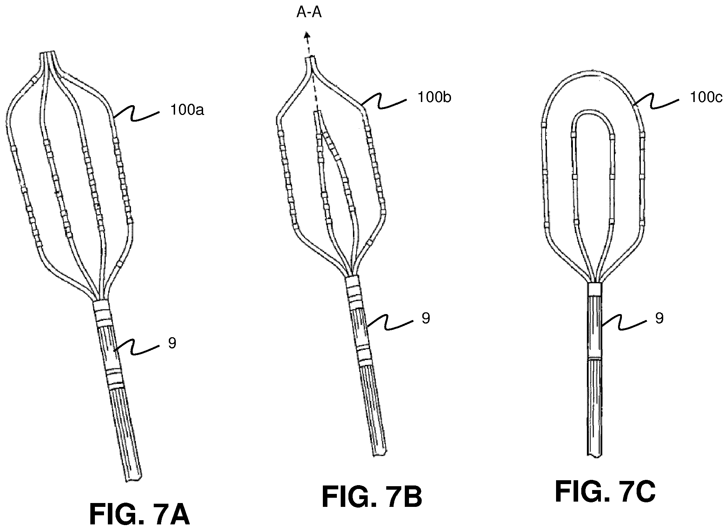

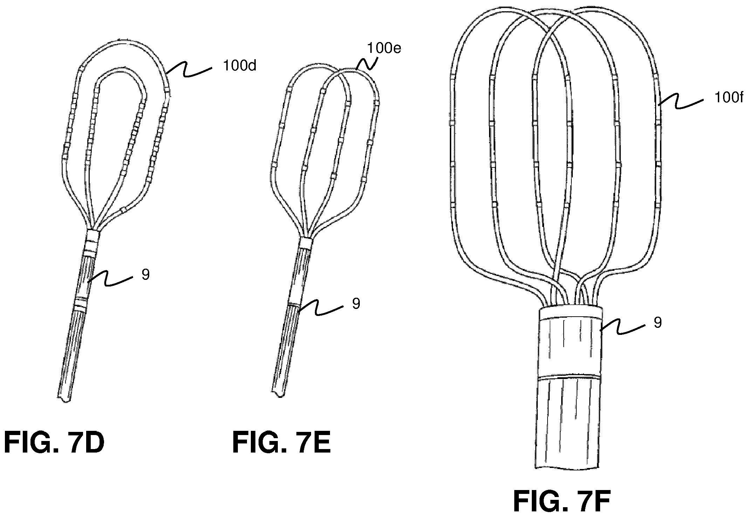

The end effector 100 is illustrated in an unconstrained configuration. When the device is unconstrained and aligned, the proximal portion 12, intermediate section 14, distal portion 14A, and end effector 100 are generally aligned along a longitudinal axis A-A. The elongated shaft 9 can define the longitudinal axis A-A of the apparatus 10. The intermediate section 14 can be configured to bend to deflect the distal portion 14A and end effector 100 from the longitudinal axis A-A similar to as described in U.S. Pat. No. 9,820,664 (see Figures 6 and 7) incorporated by reference herein in its entirety and attached in the appendix hereto. Details of the apparatus can be understood with reference to U.S. Patent Application S.N. 63/031,955.

The end effector 100 can be collapsed (compressed toward the longitudinal axis A-A) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The end effector 100 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of the first loop member 1 are connected by a first connector 1C; spines 2A, 2B of the second loop member 2 are connected by a second connector 2C; and spines 3A, 3B of the third loop member 3 are connected by a third connector 3C. Each loop member 1, 2, 3 further has a respective pair of ends affixed to the distal portion 14A of the elongated shaft 9 (e.g. affixed to the connector 46 which is an extension of the distal portion 14A of the elongated shaft 9).

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in Figure 1. Preferably, all spines in the end effector are parallel to each other along the majority of their respective lengths when the end effector 100 is in the unconstrained configuration. Even when all spines are parallel, the spines are not necessarily all coplanar. Details of the apparatus can be understood with reference to U.S. Patent Application S.N. 63/031,955.

Each spine 1A, 1B, 2A, 2B, 3A or 3B can have a length ranging between about 5 and 50 mm, preferably between about 10 and 35 mm, and more preferably about 28 mm. The parallel portions of each spine 1A, 1B, 2A, 2B, 3A, 3B can be spaced apart from each other by a distance ranging between about 1mm and 20 mm, preferably between about 2 and 10 mm, and more preferably about 4 mm. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes. The spine electrodes 37 can be positioned and otherwise configured to contact cardiovascular tissue. The spine electrodes 37 can be configured to receive electrical potentials from tissue that they are in contact with and/or ablate the tissue.

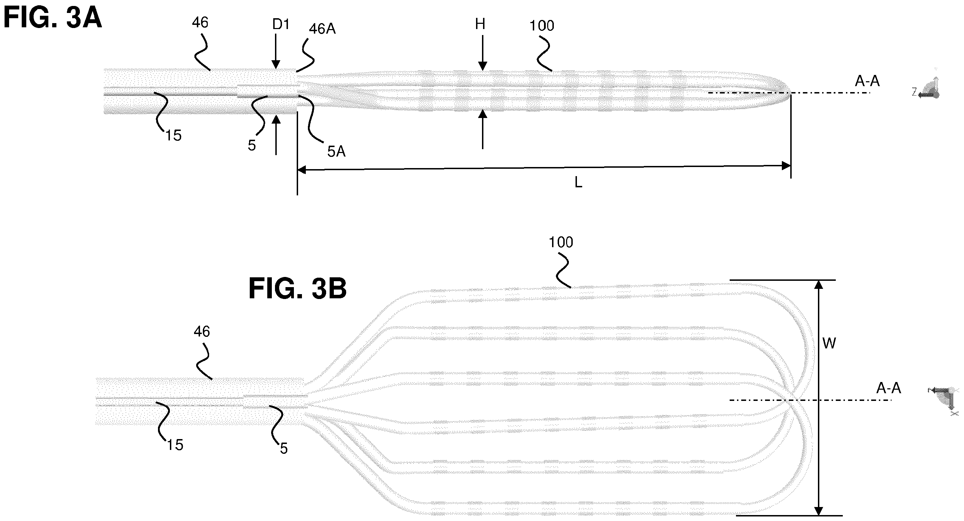

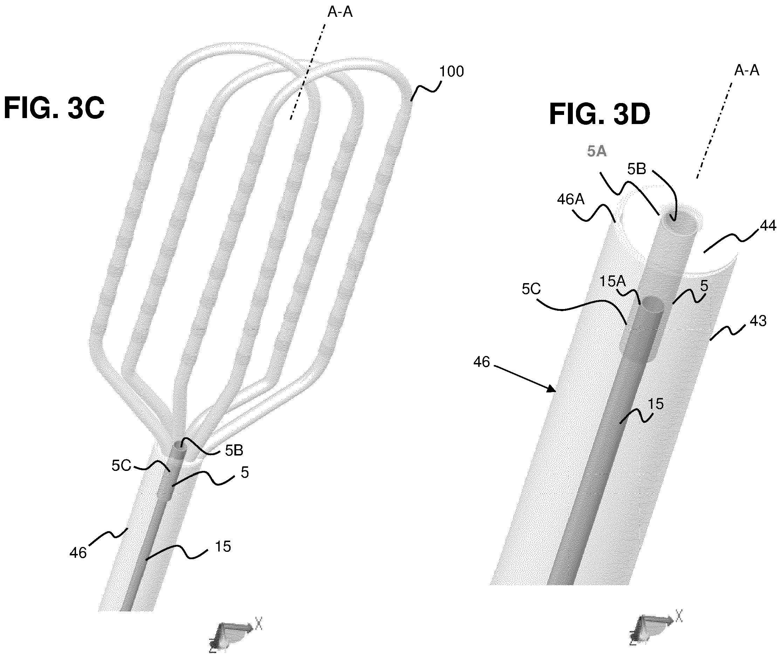

Figure 1 is illustrated as including a distal electrode 38D and a proximal electrode 38P positioned near the distal portion 14A of the shaft 9. The electrodes 38D and 38P can be configured to cooperate (e.g. by masking of a portion of one electrode and masking a different portion on the other electrode) to define a referential electrode (an electrode that is not in contact with tissues). As illustrated in greater detail in Figures 3A through 3F, the apparatus 10 can include a reference electrode 5 positioned within the connector tubing 46 so that is it is prevented from contacting tissue by the connector tubing 46. The reference electrode 5 can be used in addition to, or in place of one or both of the distal electrode 38D and the proximal electrode 38P illustrated in Figure 1.Due to the likelihood of the electrodes 38D and 38P coming into contact with tissues, it is preferable that reference electrode 5 is utilized by itself.

In some unipolar EP mapping techniques, it can be desirable to obtain a reference potential from blood near the tissue at which a tissue potential is being picked up. In other words, it may be desirable to place a first electrode in contact with tissue to thereby pick up an electrical potential from the tissue; and place a second electrode in electrical communication with blood near the contacted tissue to thereby pick up a reference electrical potential from the blood. The reference electrode can be in direct contact with the blood and/or be in electrical communication with the blood via other fluids such as irrigation fluid. The second (reference) electrode can be configured such that it is prevented from contacting tissue while maintaining electrical communication with blood. By having the second (reference) electrode avoid contact with tissue, the second (reference) electrode may avoid pickup of local tissue potentials that might otherwise compromise the reliability of the sensed reference potential. This configuration can provide benefits similar to those obtained using bipolar EP mapping devices and techniques, such as reduced noise and reduced far field signals, due to the location of the reference electrode being in the same heart chamber as tissue-contacting electrodes; while still maintaining features of a unipolar signal.

Referring again to Figure 1, it is expected that when the end effector 100 is pressed to tissue, one side of each of the reference electrodes 38D, 38P on the outside shaft 9 may contact tissue while the opposite side of each of the shaft reference electrodes 38D, 38P faces away from the tissue. The electrodes 38D, 38P can be masked with an electrical insulator on opposite sides such that when an unmasked side of one of the electrodes is against tissue, an unmasked side of the other electrode is facing away from the tissue; meaning, if one of the shaft reference electrodes 38D, 38P is in electrical contact with tissue, the other of the shaft electrodes 38D, 38P is insulated from the tissue by an electrically insulating mask.

Examples presented herein include a reference electrode 5 that has an electrically insulated outer surface such that electrically conductive portions of the reference electrode 5 are prevented from ever coming into contact with tissue. This eliminates the need for two electrodes (e.g. aforementioned shaft electrodes 38D, 38P) thereby reducing wires and bulk. The end effector 100 can be joined to the connector tubing 46 such that an outer surface area of the reference electrode 5 is partially exposed electrically and the exposed outer surface is positioned to prohibit the possibility that the exposed outer surface touches tissue during treatment.

One or more impedance sensing electrodes 38R can be configured to allow for location sensing via impedance location sensing technique, as described in U.S. Pat. Nos. 5,944,022; 5,983,126; and 6,445,864, for each of which a copy is provided in the priority U.S. Provisional Patent Application 63/052,553.

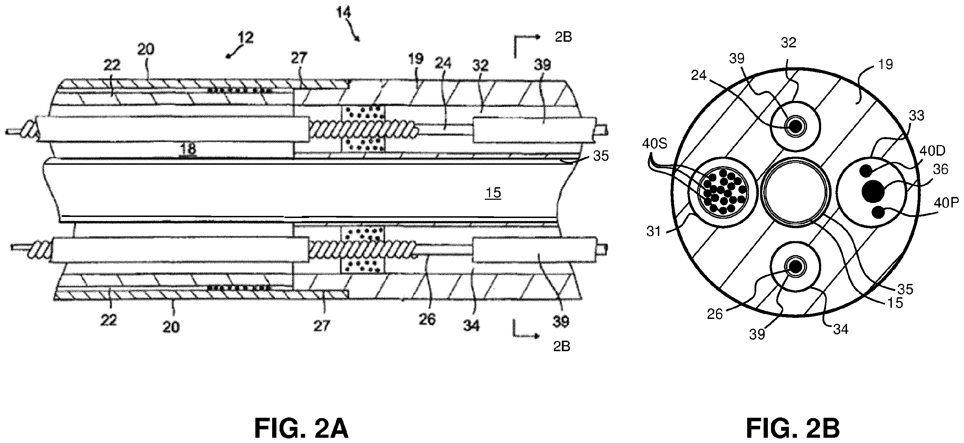

Figures 2A and 2B illustrate the intermediate section 14 and distal portion 14A of the shaft 9 of the apparatus in greater detail. Figure 2A is a cross-sectional view, along the longitudinal axis A-A, of the elongated shaft 9 at the interface between the proximal portion 12 and intermediate section 14. Figure 2B is a cross-sectional view of the intermediate section 14 orthogonal to the longitudinal axis A-A.

As illustrated in Figure 2A, the catheter body 12 can be an elongated tubular construction having a single axial passage or central lumen 18. The central lumen 18 can be sized to allow an irrigation tube 15 to pass therethrough. The catheter body 12 is flexible, i.e., bendable, but substantially non-compressible along its length. The catheter body 12 can be of any suitable construction and made of any suitable material. In some embodiments, the catheter body 12 has an outer wall 20 made of polyurethane or PEBAX. The outer wall 20 may include an imbedded braided mesh of stainless steel or the like to increase torsional stiffness of the catheter body 12 so that, when the control handle 16 is rotated, the intermediate section 14 will rotate in a corresponding manner.

The outer diameter of the catheter body 12 is preferably no more than about 8 French, more preferably about 7 French. The thickness of the outer wall 20 is thin enough so that the central lumen 18 can accommodate at least one puller wire, one or more lead wires, and any other desired wires, cables or tubes (e.g. irrigation tube 15). If desired, the inner surface of the outer wall 20 is lined with a stiffening tube 22 to provide improved torsional stability. In some embodiments, the outer wall 20 has an outer diameter of from about 0.090 inch to about 0.094 inch (from about 2.3 mm to about 2.4 mm) and an inner diameter of from about 0.061 inch to about 0.065 inch (from about 1.5 mm to about 1.7 mm).

As illustrated particularly in Figure 2B, the intermediate section 14 can include a shorter section of tubing 19 having multiple lumens, for example, four off-axis lumens 31, 32, 33, 34 and a central lumen 35. The first lumen 31 carries a plurality of lead wires 40S for ring electrodes 37 carried on the spines 1A, 1B, 2A, 2B, 3A, 3B. The second lumen 32 carries a first puller wire 24. The third lumen 33 carries a cable 36 for an electromagnetic position sensor 42 and lead wires 40D and 40P for distal and proximal ring electrodes 38D and 38P carried on the catheter proximally of the end effector 100 and/or the reference electrode 5 within the tubing 46.

Electromagnetic location sensing technique is described in U.S. Patent Nos. 5,391,199; 5,443,489; 5,558,091; 6,172,499; 6,590,963; and 6,788,967, for each of which a copy is provided in the priority U.S. Provisional Patent Application 63/052,553 . The magnetic location sensor 42 can be utilized with impedance sensing electrode 38R in a hybrid magnetic and impedance position sensing technique known as ACL described in US Patent Nos. 7,536,218; 7,756,567; 7,848,787; 7,869,865; and 8,456,182, for each of which a copy is provided in the priority U.S. Provisional Patent Application 63/052,553.

The fourth lumen 34 (for example, diametrically opposite of the second lumen 32 as illustrated) carries a second puller wire 26. The fifth, central lumen 35 carries the irrigation tube 15.

The tubing 19 is made of a suitable non-toxic material that is preferably more flexible than the catheter body 12. One suitable material for the tubing 19 is braided polyurethane, i.e., polyurethane with an embedded mesh of braided stainless steel or the like. The size of each lumen is sufficient to house the lead wires, puller wires, the cable and any other components.

The useful length of the catheter shaft 9, i.e., that portion of the apparatus 10 that can be inserted into the body excluding the end effector 100, can vary as desired. Preferably the useful length ranges from about 110 cm to about 120 cm. The length of the intermediate section 14 (measured from the connection to the catheter body 12 to the distal end of the shaft 9) is a relatively smaller portion of the useful length, and preferably ranges from about 3.5 cm to about 10 cm, more preferably from about 5 cm to about 6.5 cm.

Catheter body proximal portion 12 can be attached to the intermediate section 14 similar to as shown and described in Figures 2A and 2B of U.S. Pat. No. 9,820,664, of which a copy is provided in the priority U.S. Provisional Patent Application 63/052,553 . If desired, a spacer (not shown) can be located within the catheter body 12 between the distal end of the stiffening tube (if provided) and the proximal end of the intermediate section 14. The spacer can provide a transition in flexibility at the junction of the catheter body 12 and intermediate section 14, which can allow this junction to bend smoothly without folding or kinking. A catheter having such a spacer is described in U.S. Pat. No. 5,964,757, of which a copy is provided in the priority U.S. Provisional Patent Application 63/052,553.

The distal portion 14A of the shaft 9 can be substantially contiguous with the intermediate section 14 such that the intermediate section comprises the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14. The connector tubing 46 can also be considered a distal extension of the shaft 9 so that it is understood that the connector tubing 46 is included in the distal portion 14A of the shaft.

Figures 3A through 3C illustrate the end effector and distal portion of the shaft 9 (connector tubing 46) in various orientations where the connector tubing is illustrated as translucent and all components within the connector tubing are made invisible with the exception of the reference electrode 5 and the irrigation tube 15 for the purposes of illustration. The connector tubing 46 can further house ends of support frames which extend through the loop members 1, 2, 3 to secure the loop members 1, 2, 3 to the shaft 9 similar to the apparatus described and illustrated in U.S. Patent Application S.N. 63/031,955. The connector tubing 46 can further house electrical conductors 36, 40D, 40P, 40S such as those illustrated in Figures 2A and 2B.

Figures 3D through 3F illustrate the connector tubing in various orientations with the end effector 100 made invisible for the purpose of illustration.

A Cartesian x, y, z axis is illustrated in each of the Figures 3A through 3F to illustrate relative orientations for each of the illustrations.

Referring collectively to Figures 3A through 3F, the reference electrode 5 can include a tubular inner surface 5B and a tubular outer surface 5C. The inner tubular surface 5B can be electrically conductive. The inner surface 5B can be positioned and otherwise configured so that fluids near cardiovascular tissue (e.g. blood and/or irrigation fluid) are in contact with the inner surface 5B when the spine electrodes 37 are in contact with cardiovascular tissue. The inner surface 5B can receive electrical potentials from these fluids so that the electrical potentials received by the reference electrode act as a referential signal for the electrical potentials of the electrodes 37 carried by the spines 1A, 1B, 2A, 2B, 3A, 3B.

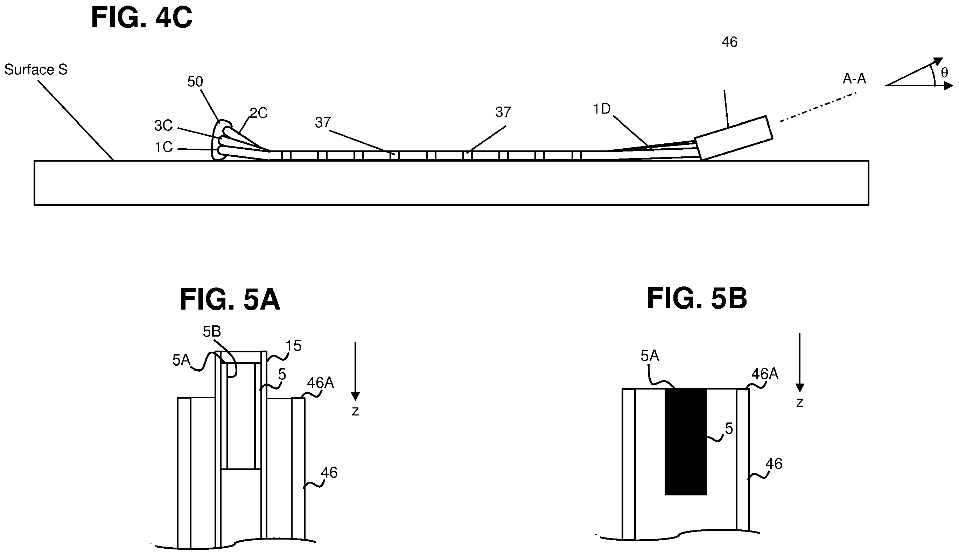

The reference electrode 5 can be disposed in the elongated shaft 9 (being disposed in the connector 46 which is a distal extension of the shaft 9) such that the electrode does not protrude beyond the elongated shaft 9. The outer surface 5C of the reference electrode 5 can be substantially electrically isolated from the electrical potentials of the fluids. The connector 46 of the shaft 9 can serve as an insulating cover which electrically isolates the outer surface 5C. The reference electrode 5 can have a distal end 5A that is approximately coaxial or coplanar with a distal end 46A of the connector 46 (which corresponds to a distal end of the shaft 9). The term "coplanar" as used in relation to the reference electrode distal end 5A and the distal end 46A means that a plane may intersect both 5A and 46A so that both ends (5A and 46A) are on the same plane. In the example shown in Fig. 3D, a virtual plane (not shown) intersects surface 5C and 46A orthogonally so that both ends 5A and 46A are on the same plane. The term "coaxial" indicates that the longitudinal axis of connector 46 and the longitudinal axis of the reference electrode 5 are coincident with each other. The connector 46 can be capable of contacting the cardiovascular tissue while the spine electrodes are in contact with the cardiovascular tissue and while the reference electrode 5 is electrically insulated, by the connector, from the cardiovascular tissue as illustrated in greater detail in Figure 4C.

The irrigation tube can be in fluidic communication with the inner surface 5B of the reference electrode 5. Fluids can flow through the irrigation tube and out of the shaft 9 through the reference electrode 5 so that the reference electrode 5 serves as a distal extension of the irrigation tube 15.

As illustrated in Figures 3A and 3B, the end effector 100 can have an unconstrained configuration aligned with a longitudinal axis A-A of the apparatus 10 and a flattened or paddle shaped geometry in the unconstrained configuration. The end effector 100 can have a height H and a width W measured as indicated in Figures 3A and 3B, the height H being significantly smaller than the width W. Further, compared to the dimensions of the connector 46, the height H of the end effector 100 can be about equal to, or smaller than, a diameter D1 of a tubular outer surface of the connector 46, where the diameter D1 is measured as indicated in Figure 3A. The width W of the end effector 100 can be substantially greater than the diameter D1 of the connector 46. The end effector can have a length L measured as indicated in Figure 3A that is greater still than the width W.

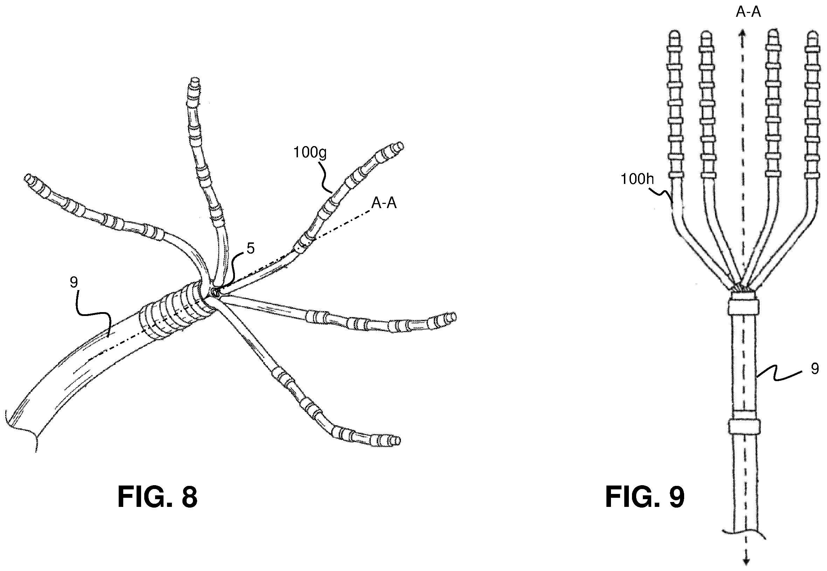

Having the paddle shape, the spine electrodes 37 are all positioned in a distal direction in relation to the reference electrode 5. When pressed to a surface, the spine electrodes 37 and references electrode 5 can roughly maintain their relative positions to each other compared to the unconstrained configuration. This results in the spine electrodes 37 being positioned to preferably be distributed in a radially asymmetric pattern on the cardiovascular tissue in relation to the reference electrode's position when the plurality of spine electrodes 37 are in contact with the cardiovascular tissue. In other words, the reference electrode 5 is positioned to one side of the collection of spine electrodes 37 when the electrodes 5, 37 are in operation during a treatment. This in contrast to other end effector geometries, such as illustrated in Figure 8 where spines of the end effector extend radially from the reference electrode 5 so that it is possible to preferably distribute spine electrodes in a radially symmetric pattern on the cardiovascular tissue in relation to the reference electrode's position. Paddle shaped end effectors such as illustrated in Figures 7A through 7F also include spine electrodes positioned to be distributed in a radially asymmetric pattern on tissue in relation to the reference electrode's position.

The spine electrodes 37 can be positioned to form a rectangular grid when the spine electrodes 37 are contact with the cardiovascular tissue. Alternatively, the spine electrodes 37 can be positioned to form a non-rectangular grid; e.g. a circular, triangular, or other such shape.

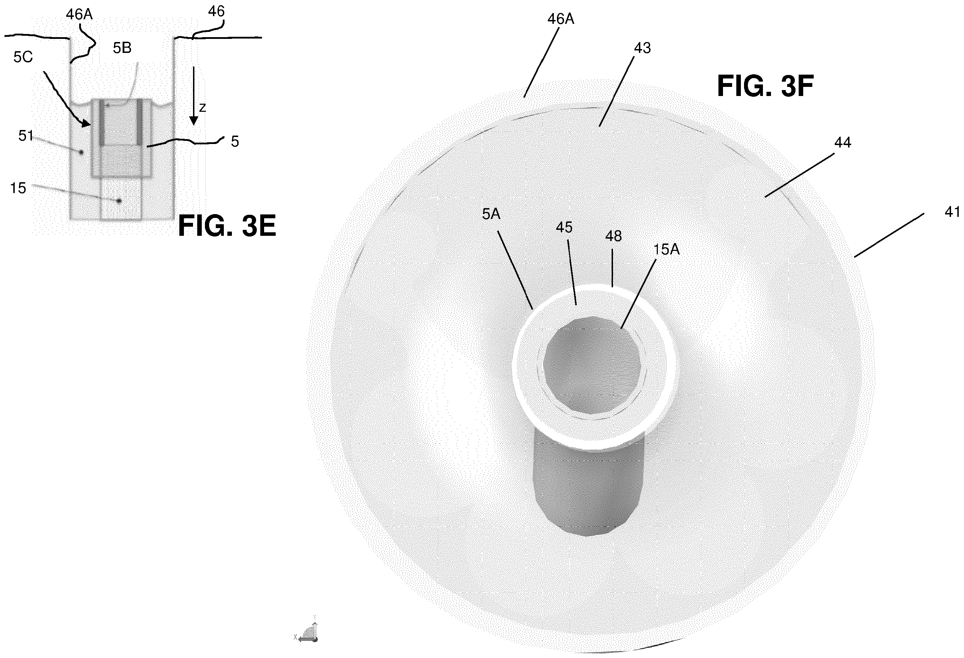

As illustrated in greater detail in Figures 3D and 3E, the irrigation tube 15 can be positioned within a lumen of the reference 5 electrode. The apparatus 10 can further include an irrigation tube cover 45 to act as a fluid impermeable seal between the reference electrode 5 and the irrigation tube 15.

The distal end 5A of the electrode 5 is approximately co-planar with the distal end 46A of the connector 46 or even recessed (Fig. 3E) so that the electrode 5 does not protrude from the shaft 9 (e.g., Fig. 1). An insulating material 51 can be provided between the outer surface 5C of the electrode 5 and the inside surface 46B of connector 46. In one embodiment, material 51 can be a polymer such as for example, polyurethane. Configured as such, insulating material 51 insulates the outer surface 5C of the electrode 5 to prevent the outer surface 5C from coming into electrical contact with tissue during use. At the same time, however, inner surface 5C of reference electrode 5 is able to have electrical and physical contact with the biological fluid in the organ (e.g., blood) in order to receive or record the electrical signals propagated in the fluid. It is noted that irrigation line 15 can also work in reverse, by aspirating or sucking blood from the organ into the irrigation line 15. This allows for the reference electrode 5 to receive or sense electrical signals (via wiring 5D or electrical trace) in the blood significantly better because the blood is pulled into irrigation tube 15 allowing immersion of the conductive inner surface 5C with the blood pulled into irrigation tube 15.

As illustrated in greater detail in Figure 3F, the connector 46 can include a collar 41 forming the outer surface 46A of the connector 46 and an insert 43 positioned in the collar 41. The insert 43 can include openings 44 shaped to receive ends of the loop members 1, 2, 3 and a central lumen 48 sized to house the reference electrode 45. A polymer can be flowed into the collar 41 and insert 43 to help secure the loop members 1, 2, 3 and reference electrode 5 in the connector 46.

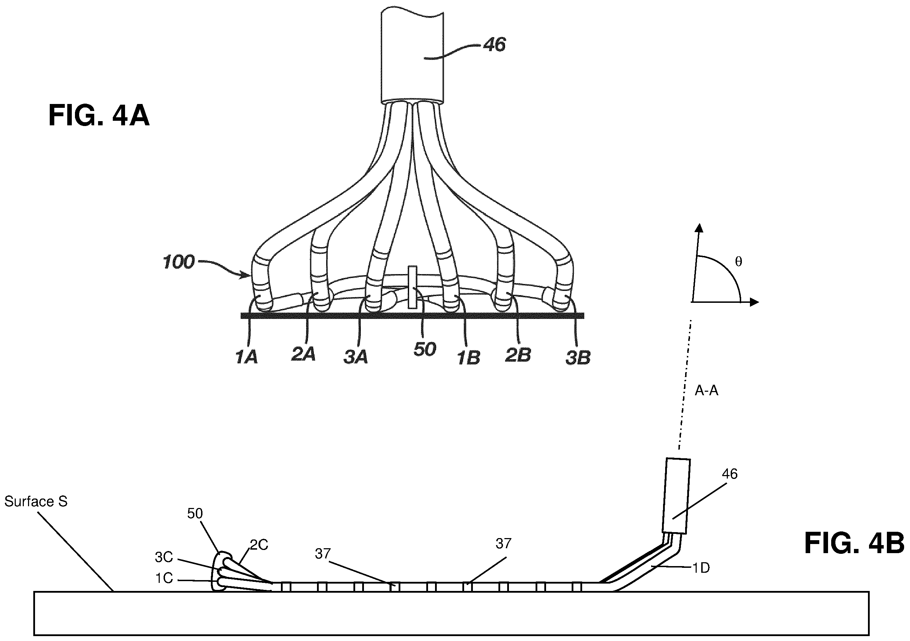

Figures 4A through 4C are illustrations of the end effector 100 being pressed to a surface S. The end effector 100 can deflect at an angle θ relative to the longitudinal axis A-A to position the spine electrodes 37 to the surface S. The surface S is illustrated as planar; however, the surface can be curved. For instance, the surface S can have curvature consistent with intracardiac tissue surfaces.

Figure 4A illustrates the end effector 100 having a portion of spine electrodes 37 pressed to the surface S and another portion of the spine electrodes 37 above the surface S, not in contact with the surface S.

Figure 4B illustrates the connector 46 positioned above the surface S with all spine electrodes 37 in contact with the surface. The end effector 100 is deflected an angle θ relative to the longitudinal axis A-A that is nearly 90°. At such an angle, were the reference electrode 5 positioned such that it protruded from the shaft 9, depending on the length of protrusion and the distance between the distal end 46A of the connector 46 and the surface S, the reference electrode 5 may come into contact with the surface S. If the reference electrode 5 were to make electrical contact with the surface S, it can reduce the effectiveness of the reference electrode's ability to sense electrical potentials that can serve as reference electrical potentials. In other words, were the reference electrode 5 to come into electrical contact with tissue, it may not function as a reliable reference electrode. Positioning the reference electrode 5 so that it does not protrude from the connector 46 of the shaft 9 prevents the reference electrode 5 from contacting the surface S even when the end effector 100 is deflected at an angle of about 90° from the longitudinal axis A-A of the shaft 9.

Figure 4C illustrates the connector 46 in contact with the surface S with all spine electrodes 37 in contact with the surface S. The end effector 100 is deflected at an acute angle θ relative to the longitudinal axis A-A. Having the connector 46 very near to the surface S and at a non-zero angle to the surface S, were the reference electrode 5 positioned such that it protruded from the shaft 9, depending on the length of protrusion and the angle θ, the reference electrode 5 may come into contact with the surface S. Positioning the reference electrode 5 so that it does not protrude from the connector 46 of the shaft 9 prevents the reference electrode 5 from contacting the surface S even when the connector 46 is in contact with the surface S and angled toward the surface S.

Figures 5A and 5B are illustrations of an alternative reference electrode configuration. The figures include a z axis to provide orientation relative to Figures 3A through 3F.

Figure 5A is an illustration of a cross section of an alternative reference electrode configuration. Only the connector 46, reference electrode 5, and irrigation tube 5 are illustrated for the purposes of illustration. The apparatus 10 can include additional components not illustrated such as conductors and mechanical connection to the end effector 100 described elsewhere herein, including the Appendix. The irrigation tube 15 can be positioned around the outer surface 5C of the reference electrode 5. The transition from the irrigation tube 15 to the reference electrode 5 can be sealed to cause the reference electrode 5 to be an extension of the irrigation tube 15. In addition, or as an alternative to the outer surface 5C being electrically insulated by the connector 46, the outer surface 5C of the reference electrode 5 can be electrically insulated by a portion of the irrigation tube 15 positioned over the outer surface 5C of the reference electrode 5. In some configurations, such as illustrated, the irrigation tube 15 and reference electrode 5 can protrude from the elongated shaft so that the irrigation tube 15 prevents the outer surface 5C of the reference electrode 5 from coming into contact with tissue. For instance, the distal end 5A of the reference electrode 5 can be positioned in the irrigation tube 15 so that the reference electrode 5 does not protrude from the irrigation tube 15.

Figure 5B is an illustration of a cross section of another alternative reference electrode configuration. In the alternative configuration, the reference electrode does not serve as an extension to the irrigation tube 15 but rather has an electrically conductive distal surface at its distal end 5A that is configured to receive electrical potentials from fluids that act as a reference signal for the electrical potentials from the spine electrodes 37. The electrically conductive surface at the distal end 5A of the reference electrode 5 is approximately coplanar or coaxial to the distal end 46A of the connector 46. Only the connector 46 and reference electrode 5 are illustrated for the purposes of illustration. The apparatus 10 can include additional components not illustrated such as conductors and mechanical connection to the end effector 100 described elsewhere herein, including in the Appendix.

Figure 6 is an illustration of an exemplary medical procedure and associated components of a cardiac electrophysiology (EP) mapping catheter system that may utilize the apparatus 10 (also referred to as a catheter assembly). A physician PH is illustrated grasping the handle 16 of the apparatus, with the end effector 100 (not shown in Figure 6) disposed in a patient PA to perform EP mapping in or near the heart H of the patient PA. The apparatus 10 is coupled with a guidance and drive system 110 via a cable 130. The apparatus 10 can optionally be coupled with a fluid source 142 via a fluid conduit 140. A set of field generators 120 are positioned underneath the patient PA and are coupled with guidance and drive system 110 via another cable 122. Field generators 120 are also optional.

The guidance and drive system 110 can include a console 112 and a display 118. The console 112 can include a first driver module 114 and a second driver module 116. The first driver module 114 can be coupled with the apparatus via a cable 130. In some variations, the first driver module 114 is operable to receive EP mapping signals obtained via spine electrodes 37 of end effector 100. The console 112 can include a processor (not shown) that processes such EP mapping signals and thereby provides EP mapping. In addition, or in the alternative, the first driver module 114 may be operable to provide RF power to the spine electrodes 37 of end effector 100 to thereby ablate tissue. In some versions, the first driver module 114 is also operable to receive position indicative signals from a position sensor in end effector 100. In such versions, the processor of console 112 is also operable to process the position indicative signals from the position sensor to thereby determine the position of the end effector 100 within the patient PA.

The guidance and drive system 110 can further include non-transitory computer readable medium with instructions thereon to case the drive system 110 to perform functionality described herein and/or as are known related to use of similar equipment. In some examples, the non-transitory computer readable memory can be in communication with the first driver module 114 (e.g. by virtue of being in communication with a processor of the first driver module 114 and/or the processor of the console 112). The non-transitory computer readable medium can include instructions thereon that when executed by the first driver module 114 cause the first driver module 114 to receive EP mapping signals from the spine electrodes 37 and a reference signal from the reference electrode 5.

The second driver module 116 is coupled with field generators 120 via a cable 122. The second driver module 116 is operable to activate field generators 120 to generate an alternating magnetic field around the heart H of the patient PA. For instance, the field generators 120 may include coils that generate alternating magnetic fields in a predetermined working volume that contains the heart H.

Some versions of the apparatus 10 include a position sense near or within the end effector 100 that is operable to generate signals that are indicative of the position and orientation of end effector 100 within the patient PA. Each position sensor may include a wire coil or a plurality of wire coils (e.g., three orthogonal coils) that are configured to generate electrical signals in response to the presence of an alternating electromagnetic field generated by field generators 120. Other components and techniques that may be used to generate real-time position data associated with end effector 100 may include wireless triangulation, acoustic tracking, optical tracking, inertial tracking, and the like. By way of example only, position sensing may be provided in accordance with at least some of the teachings of U.S. Pat. No. 9,480,416, of which a copy is provided in the priority U.S. Provisional Patent Application 63/052,553. Alternatively, apparatus 10 may lack a position sensor near the end effector 100.

The display 118 is coupled with the processor of console 112 and is operable to render images of patient anatomy. Such images may be based on a set of preoperatively or intraoperatively obtained images (e.g., a CT or MRI scan, 3-D map, etc.). The views of patient anatomy provided through the display 118 may also change dynamically based on signals from the position sensor near the end effector 100.

The processor of the console 112 may also drive the display 118 to superimpose the current location of end effector 100 on the images of the patient's anatomy, such as by superimposing an illuminated dot, a crosshair, a graphical representation of end effector 100, or some other form of visual indication.

The fluid source 142 can include a bag containing saline or some other suitable irrigation fluid. The conduit 140 can include a flexible tube that is further coupled with a pump 144, which is operable to selectively drive fluid from the fluid source 142 to the irrigation tube 15 of the apparatus 10. In some variations, such as including a reference electrode 5 as configured in Figure 5B, the conduit 140, fluid source 142, and pump 144 are omitted entirely.

Figures 7A through 7F are illustrations of alternative end effectors 100a-f having loop members and including an intralumenal reference electrode 5 described elsewhere herein. The end effectors include a reference electrode that does not protrude from the shaft 9 and therefore is not visible in the views of the figures. The end effectors can otherwise be configured similarly to the end effectors illustrated in U.S. Pat. No. 9,820,664 (see Figures 8A through 8F), U.S. Pub. No. 2020/0038101, and U.S. Pub. No. 2020/0038101 (see Figures 10 through 15), for each of which a copy is provided in the priority U.S. Provisional Patent Application 63/052,553.

Figure 8 is an illustration of an alternative end effector 100g having a ray geometry and including an intralumenal reference electrode according to aspects of the present invention. The end effector includes a reference electrode 5 that does not extend beyond the shaft 9. The end effector can otherwise be configured similarly to the end effectors illustrated in U.S. Pat. No. 9,907,480 (see Figure 5), U.S. Pub. No. 2018/0056038 (see Figure 5), and U.S. Pub. No. 2020/0038101 (see Figures 2 through 7, 19, and 20). It is noted that the reference electrode as described and illustrated herein can be utilized with an end effector having a basket configuration whereby each of the spines expand outward and converges towards a distal end is within the scope of the invention. One example of such basket configuration is shown exemplarily in U.S. Pat. No. 10,575,743B2.

Figure 9 is an illustration of another alternative end effector 100h including an intralumenal reference electrode according to aspects of the present invention. The end effectors include a reference electrode that does not protrude from the shaft 9 and therefore is not visible in the views of the figures. The end effector can otherwise be configured similarly to the end effectors illustrated in U.S. Pat. No. 9,820,664 (see Figures 5A through 7) and U.S. Pub. No. 2020/0038101 (see Figure 16).

## Claims

1. An apparatus comprising:
an elongated shaft (9) comprising a proximal portion (12) and a distal portion (14A), the elongated shaft configured to be manipulated at the proximal portion to position the distal portion into a heart of a patient;
an end effector (100) disposed proximate the distal portion of the elongated shaft and comprising spines (1A, 1B, 2A, 2B, 3A, 3B) each carrying at least one spine electrode (37) configured to contact cardiovascular tissue and receive electrical potentials from the tissue; and
a reference electrode (5) comprising an inner surface (5B) and an outer surface (5C) disposed about a longitudinal axis extending along the elongated shaft (9), the reference electrode (5) being disposed in the elongated shaft (9) such that the reference electrode (5) does not protrude beyond the elongated shaft (9),
the inner surface (5B) of the reference electrode (5) being configured to receive electrical potentials from fluids that act as a referential signal for the electrical potentials of the at least one spine electrode (37), and
the outer surface of the reference electrode being electrically isolated from the electrical potentials of the fluids.

2. The apparatus of claim 1, further comprising:
a connector (46) joining the elongated shaft (9) to the end effector, the connector (46) comprising the electrically insulating cover.

3. The apparatus of claim 2, the reference electrode comprising a distal end approximately coplanar to a distal end of the connector.

4. The apparatus of claim 2, the connector being capable of contacting the cardiovascular tissue while the spine electrodes are in contact with the cardiovascular tissue and the outer surface of the reference electrode is electrically insulated, by the connector, from the cardiovascular tissue.

5. The apparatus of claim 2,
the end effector comprising an unconstrained configuration aligned with a longitudinal axis of the apparatus,
the connector comprising a tubular outer surface comprising a diameter measured orthogonal to the longitudinal axis,
the end effector comprising a height measured orthogonal to the longitudinal axis and a width measured orthogonal to the height and longitudinal axis when the end effector is in the unconstrained configuration,
the height measuring approximately equal to or less than the diameter of the tubular outer surface of the connector, and
the width measuring greater than the diameter of the tubular outer surface of the connector.

6. The apparatus of claim 1, further comprising:
an irrigation tube (15) in fluidic communication with the inner surface of the reference electrode.

7. The apparatus of claim 6, the irrigation tube being configured in one mode to move irrigation fluid out of the irrigation tube and in another mode to move body fluids from an organ into the irrigation tube to allow for contact of the body fluids with the inner surface of the reference electrode.

8. The apparatus of claim 1, the spine electrodes positioned to form a grid when the spine electrodes are configured to contact with the cardiovascular tissue.

9. The apparatus of claim 1,
the elongated shaft defining a longitudinal axis of the apparatus,
the end effector comprising three loop members (1, 2, 3) each comprising at least one of the spines and joined at a common distal vertex (50) along the longitudinal axis,
each of the three loop members comprising a respective pair of ends affixed to the distal portion of the elongated shaft, and
the end effector configured to deflect at an angle relative to the longitudinal axis to position the spine electrodes against a planar surface.

10. The apparatus of claim 1, the spine electrodes being configured to receive electrical potentials from the cardiovascular tissue when in contact with the cardiovascular tissue, or to ablate when in contact with the cardiovascular tissue.

11. The apparatus of claim 1, the reference electrode comprising a cylindrical member disposed about the longitudinal axis with the inner surface of the cylindrical member configured to contact with body fluids when placed into an organ.

12. A method comprising:
assembling an end effector (100) such that the end effector comprises at least one spine electrode (37) thereon configured to contact cardiovascular tissue and receive electrical potentials from the tissue;
affixing a reference electrode (5) in relation to the end effector such that a tubular inner surface (5B) of the reference electrode (5) does not contact cardiovascular tissue and is configured to receive electrical potentials from fluids that act as a referential signal for the electrical potentials received by the electrodes;
electrically insulating an outer surface (5C) of the reference electrode (5) such that the outer surface (5C) is electrically isolated from the electrical potentials from fluids approximate the cardiovascular tissue while the inner surface (5B) is capable of receiving the electrical potentials of the fluids; and
affixing the end effector (100) and reference electrode (5) to a distal portion (14A) of an elongated shaft (9), the elongated shaft configured to be manipulated at a proximal portion (12) of the elongated shaft (9) to position the distal portion into a heart of a patient.

13. The method of claim 12, further comprising;
joining the elongated shaft to the end effector with a connector; and
electrically insulating a portion of the outer surface of the reference electrode with the connector so that an electrically exposed portion of the outer surface is positioned to preclude the exposed portion from coming into contact with tissue during treatment.

14. The method of claim 13, further comprising:
affixing the reference electrode and the connector such that a distal end of the reference electrode and a distal end of the connector are approximately coplanar and coaxial to each other.

15. The method of claim 13, further comprising:
joining the elongated shaft to the end effector such that the connector is capable of contacting the cardiovascular tissue while the spine electrode is in contact with the cardiovascular tissue and while the reference electrode is electrically insulated, by the connector, from the cardiovascular tissue.

16. The method of claim 13, further comprising:
positioning an irrigation tube within the elongated shaft such that the irrigation tube is in fluidic communication with the inner surface of the reference electrode.

## Patentansprüche

1. Einrichtung, umfassend:
einen länglichen Schaft (9) mit einem proximalen Abschnitt (12) und einem distalen Abschnitt (14A), wobei der längliche Schaft dafür ausgelegt ist, am proximalen Abschnitt gehandhabt zu werden, um den distalen Abschnitt in ein Herz eines Patienten zu positionieren;
einen Endeffektor (100), der in der Nähe des distalen Abschnitts des länglichen Schafts angeordnet ist und Röhren (1A, 1B, 2A, 2B, 3A, 3B) umfasst, die jeweils wenigstens eine Röhrenelektrode (37) tragen, die dafür ausgelegt ist, mit kardiovaskulärem Gewebe in Kontakt zu kommen und elektrische Potenziale von dem Gewebe aufzunehmen; und
eine Referenzelektrode (5), die eine Innenfläche (5B) und eine Außenfläche (5C) umfasst, die um eine Längsachse angeordnet sind, die sich entlang des länglichen Schafts (9) erstreckt, wobei die Referenzelektrode (5) derart im länglichen Schaft (9) angeordnet ist, dass die Referenzelektrode (5) nicht aus dem länglichen Schaft (9) herausragt,
wobei die Innenfläche (5B) der Referenzelektrode (5) dafür ausgelegt ist, elektrische Potentiale von Flüssigkeiten aufzunehmen, die als Referenzsignal für die elektrischen Potentiale der wenigstens einen Röhrenelektrode (37) dienen, und
wobei die Außenfläche der Referenzelektrode elektrisch von den elektrischen Potentialen der Flüssigkeiten isoliert ist.

2. Einrichtung nach Anspruch 1, ferner umfassend:
ein Verbindungsstück (46), das den länglichen Schaft (9) mit dem Endeffektor verbindet, wobei das Verbindungsstück (46) die elektrisch isolierende Abdeckung umfasst.

3. Einrichtung nach Anspruch 2, wobei die Referenzelektrode ein distales Ende umfasst, das ungefähr koplanar zu einem distalen Ende des Verbindungsstücks ist.

4. Einrichtung nach Anspruch 2, wobei das Verbindungsstück in der Lage ist, mit dem kardiovaskulären Gewebe in Kontakt zu kommen, während die Röhrenelektroden in Kontakt mit dem kardiovaskulären Gewebe sind und die Außenfläche der Referenzelektrode durch das Verbindungsstück elektrisch von dem kardiovaskulären Gewebe isoliert ist.

5. Einrichtung nach Anspruch 2,
wobei der Endeffektor eine uneingeschränkte Konfiguration aufweist, die an einer Längsachse der Einrichtung ausgerichtet ist,
wobei das Verbindungsstück eine röhrenförmige Außenfläche aufweist, die einen orthogonal zur Längsachse gemessenen Durchmesser umfasst,
wobei der Endeffektor eine orthogonal zur Längsachse gemessene Höhe und eine orthogonal zur Höhe und Längsachse gemessene Breite umfasst, wenn sich der Endeffektor in der uneingeschränkten Konfiguration befindet,
wobei der Höhenmesswert ungefähr gleich oder kleiner als der Durchmesser der röhrenförmigen Außenfläche des Verbindungsstücks ist und
wobei der Breitenmesswert größer als der Durchmesser der röhrenförmigen Außenfläche des Verbindungsstücks ist.

6. Einrichtung nach Anspruch 1, ferner umfassend:
einen Spülschlauch (15), der mit der Innenfläche der Referenzelektrode in Fluidverbindung steht.

7. Einrichtung nach Anspruch 6, wobei der Spülschlauch dafür ausgelegt ist, in einem Modus Spülflüssigkeit aus dem Spülschlauch zu transportieren und in einem anderen Modus Körperflüssigkeiten aus einem Organ in den Spülschlauch zu transportieren, um einen Kontakt der Körperflüssigkeiten mit der Innenfläche der Referenzelektrode zu ermöglichen.

8. Einrichtung nach Anspruch 1, wobei die Röhrenelektroden so positioniert sind, dass sie ein Gitter bilden, wenn die Röhrenelektroden dafür ausgelegt sind, mit dem kardiovaskulären Gewebe in Kontakt zu kommen.

9. Einrichtung nach Anspruch 1,
wobei der längliche Schaft eine Längsachse der Einrichtung definiert,
wobei der Endeffektor drei Schlaufenelemente (1, 2, 3) umfasst, die jeweils wenigstens eine der Röhren aufweisen und an einem gemeinsamen distalen Scheitelpunkt (50) entlang der Längsachse verbunden sind,
wobei jedes der drei Schlaufenelemente ein jeweiliges Paar von Enden umfasst, die am distalen Abschnitt des länglichen Schafts befestigt sind, und
wobei der Endeffektor dafür ausgelegt ist, in einem Winkel relativ zur Längsachse gebogen zu werden, um die Röhrenelektroden gegen eine ebene Fläche zu positionieren.

10. Einrichtung nach Anspruch 1, wobei die Röhrenelektroden dafür ausgelegt sind, elektrische Potentiale vom kardiovaskulären Gewebe aufzunehmen, wenn sie in Kontakt mit dem kardiovaskulären Gewebe sind, oder zu ablatieren, wenn sie in Kontakt mit dem kardiovaskulären Gewebe sind.

11. Einrichtung nach Anspruch 1, wobei die Referenzelektrode ein zylindrisches Element umfasst, das um die Längsachse angeordnet ist, wobei die Innenfläche des zylindrischen Elements dafür ausgelegt ist, mit Körperflüssigkeiten in Kontakt zu kommen, wenn sie in ein Organ eingeführt wird.

12. Verfahren, umfassend:
Montieren eines Endeffektors (100) derart, dass der Endeffektor wenigstens eine Röhrenelektrode (37) darauf umfasst, die dafür ausgelegt ist, mit kardiovaskulärem Gewebe in Kontakt zu kommen und elektrische Potentiale von dem Gewebe aufzunehmen;
Anbringen einer Referenzelektrode (5) relativ zum Endeffektor, etwa derart, dass eine röhrenförmige Innenfläche (5B) der Referenzelektrode (5) nicht mit kardiovaskulärem Gewebe in Kontakt kommt und dafür ausgelegt ist, elektrische Potentiale von Flüssigkeiten aufzunehmen, die als Referenzsignal für die durch die Elektroden empfangenen elektrischen Potentiale dienen;
elektrisches Isolieren einer Außenfläche (5C) der Referenzelektrode (5), etwa derart, dass die Außenfläche (5C) von den elektrischen Potenzialen von Flüssigkeiten in der Umgebung des kardiovaskulären Gewebes elektrisch isoliert ist, während die Innenfläche (5B) die elektrischen Potenziale der Flüssigkeiten aufnehmen kann; und
Anbringen des Endeffektors (100) und der Referenzelektrode (5) am distalen Abschnitt (14A) eines länglichen Schafts (9), wobei der längliche Schaft dafür ausgelegt ist, an einem proximalen Abschnitt (12) des länglichen Schafts (9) gehandhabt zu werden, um den distalen Abschnitt in ein Herz eines Patienten zu positionieren.

13. Verfahren nach Anspruch 12, ferner umfassend:
Verbinden des länglichen Schafts mit dem Endeffektor mit einem Verbindungsstück; und
elektrisches Isolieren eines Abschnitts der Außenfläche der Referenzelektrode mit dem Verbindungsstück, so dass ein elektrisch exponierter Abschnitt der Außenfläche derart positioniert ist, dass der exponierte Abschnitt während der Behandlung nicht mit Gewebe in Kontakt kommen kann.

14. Verfahren nach Anspruch 13, ferner umfassend:
Befestigen der Referenzelektrode und des Verbindungsstücks etwa derart, dass ein distales Ende der Referenzelektrode und ein distales Ende des Verbindungsstücks annähernd koplanar und koaxial zueinander sind.

15. Verfahren nach Anspruch 13, ferner umfassend:
Verbinden des länglichen Schafts mit dem Endeffektor derart, dass das Verbindungsstück mit dem kardiovaskulären Gewebe in Kontakt kommen kann, während die Röhrenelektrode in Kontakt mit dem kardiovaskulären Gewebe ist und während die Referenzelektrode durch das Verbindungsstück elektrisch vom kardiovaskulären Gewebe isoliert ist.

16. Verfahren nach Anspruch 13, ferner umfassend:
Positionieren eines Spülschlauchs innerhalb des länglichen Schafts, etwa derart, dass der Spülschlauch mit der Innenfläche der Referenzelektrode in Fluidverbindung steht.

## Revendications

1. Appareil comprenant :
une tige allongée (9) comprenant une partie proximale (12) et une partie distale (14A), la tige allongée étant configurée pour être manipulé au niveau de la partie proximale pour positionner la partie distale dans un cœur d'un patient ;
un effecteur terminal (100) disposé à proximité de la partie distale de la tige allongée et comprenant des colonnes (1A, 1B, 2A, 2B, 3A, 3B) portant chacune au moins une électrode de colonne (37) configurée pour entrer en contact avec un tissu cardiovasculaire et recevoir des potentiels électriques du tissu ; et
une électrode de référence (5) comprenant une surface interne (5B) et une surface extérieure (5C) disposée autour d'un axe longitudinal s'étendant le long de la tige allongée (9), l'électrode de référence (5) étant disposée dans la tige allongée (9) de telle sorte que l'électrode de référence (5) ne fait pas saillie au-delà de la tige allongée (9),
la surface interne (5B) de l'électrode de référence (5) étant configurée pour recevoir des potentiels électriques de fluides qui agissent comme un signal de référence pour les potentiels électriques de l'au moins une électrode de colonne (37), et
la surface extérieure de l'électrode de référence étant isolée électriquement des potentiels électriques des fluides.

2. Appareil selon la revendication 1, comprenant en outre :
un connecteur (46) reliant la tige allongée (9) à l'effecteur terminal, le connecteur (46) comprenant le couvercle électriquement isolant.

3. Appareil selon la revendication 2, l'électrode de référence comprenant une extrémité distale approximativement coplanaire à une extrémité distale du connecteur.

4. Appareil selon la revendication 2, le connecteur étant capable d'entrer en contact avec le tissu cardiovasculaire alors que les électrodes de colonne sont en contact avec le tissu cardiovasculaire et que la surface extérieure de l'électrode de référence est électriquement isolée, par le connecteur, du tissu cardiovasculaire.

5. Appareil selon la revendication 2,
l'effecteur terminal comprenant une configuration non contrainte alignée avec un axe longitudinal de l'appareil,
le connecteur comprenant une surface extérieure tubulaire comprenant un diamètre mesuré orthogonalement à l'axe longitudinal,
l'effecteur terminal comprenant une hauteur mesurée orthogonalement à l'axe longitudinal et une largeur mesurée orthogonalement à la hauteur et à l'axe longitudinal lorsque l'effecteur terminal est dans la configuration non contrainte,
la hauteur étant approximativement égale ou inférieure au diamètre de la surface extérieure tubulaire du connecteur, et
la largeur étant supérieure au diamètre de la surface extérieure tubulaire du connecteur.

6. Appareil selon la revendication 1, comprenant en outre :
un tube d'irrigation (15) en communication fluidique avec la surface interne de l'électrode de référence.

7. Appareil selon la revendication 6, le tube d'irrigation étant configuré dans un mode pour déplacer le fluide d'irrigation hors du tube d'irrigation et dans un autre mode pour déplacer les fluides corporels d'un organe dans le tube d'irrigation pour permettre le contact des fluides corporels avec la surface interne de l'électrode de référence.

8. Appareil selon la revendication 1, les électrodes de colonne étant positionnées pour former une grille lorsque les électrodes de colonne sont configurées pour entrer en contact avec le tissu cardiovasculaire.

9. Appareil selon la revendication 1,
la tige allongée définissant un axe longitudinal de l'appareil,
l'effecteur terminal comprenant trois éléments de boucle (1, 2, 3) comprenant chacun au moins une des colonnes et joints à un sommet distal commun (50) le long de l'axe longitudinal,
chacun des trois éléments de boucle comprenant une paire respective d'extrémités fixées à la partie distale de la tige allongée, et
l'effecteur terminal étant configuré pour dévier selon un angle par rapport à l'axe longitudinal afin de positionner les électrodes de colonne contre une surface plane.

10. Appareil selon la revendication 1, les électrodes de colonne étant configurées pour recevoir des potentiels électriques du tissu cardiovasculaire lorsqu'elles sont en contact avec le tissu cardiovasculaire, ou pour effectuer une ablation lorsqu'elles sont en contact avec le tissu cardiovasculaire.

11. Appareil selon la revendication 1, l'électrode de référence comprenant un élément cylindrique disposé autour de l'axe longitudinal, la surface interne de l'élément cylindrique étant configurée pour entrer en contact avec des fluides corporels lorsqu'elle est placée dans un organe.

12. Procédé comprenant :
l'assemblage d'un effecteur terminal (100) de telle sorte que l'effecteur terminal comprend au moins une électrode de colonne (37) sur celui-ci configurée pour entrer en contact avec un tissu cardiovasculaire et recevoir des potentiels électriques du tissu ;
la fixation d'une électrode de référence (5) par rapport à l'effecteur terminal de telle sorte qu'une surface interne tubulaire (5B) de l'électrode de référence (5) n'entre pas en contact avec le tissu cardiovasculaire et est configurée pour recevoir des potentiels électriques provenant de fluides qui agissent comme un signal de référence pour les potentiels électriques reçus par les électrodes ;
l'isolation électriquement d'une surface extérieure (5C) de l'électrode de référence (5) de telle sorte que la surface extérieure (5C) est électriquement isolée des potentiels électriques provenant de fluides proches du tissu cardiovasculaire tandis que la surface interne (5B) est capable de recevoir les potentiels électriques des fluides ; et
la fixation de l'effecteur terminal (100) et de l'électrode de référence (5) à une partie distale (14A) d'une tige allongée (9), la tige allongée étant configurée pour être manipulée au niveau d'une partie proximale (12) de la tige allongée (9) pour positionner la partie distale dans le cœur d'un patient.

13. Procédé selon la revendication 12, comprenant en outre :
la liaison de la tige allongée à l'effecteur terminal avec un connecteur ; et
l'isolation électrique d'une partie de la surface extérieure de l'électrode de référence avec le connecteur de sorte qu'une partie électriquement exposée de la surface extérieure est positionnée pour empêcher la partie exposée d'entrer en contact avec le tissu pendant le traitement.

14. Procédé selon la revendication 13, comprenant en outre :
la fixation de l'électrode de référence et du connecteur de sorte qu'une extrémité distale de l'électrode de référence et une extrémité distale du connecteur sont approximativement coplanaires et coaxiales l'une par rapport à l'autre.

15. Procédé selon la revendication 13, comprenant en outre :
la jonction de la tige allongée à l'effecteur terminal de telle sorte que le connecteur est capable d'entrer en contact avec le tissu cardiovasculaire alors que l'électrode de colonne est en contact avec le tissu cardiovasculaire et alors que l'électrode de référence est électriquement isolée, par le connecteur, du tissu cardiovasculaire.

16. Procédé selon la revendication 13, comprenant en outre :
le positionnement d'un tube d'irrigation à l'intérieur de la tige allongée de telle sorte que le tube d'irrigation est en communication fluidique avec la surface interne de l'électrode de référence.
